# EUROPEAN PATENT SPECIFICATION

(11) **EP 0 488 403 B1**
(45) Date of publication and mention of the grant of the patent: **25.10.1995**
(21) Application number: 91120589.6
(22) Date of filing: 29.11.1991
(51) Int. Cl.: C07B 61/02, C07D 211/94, C07D 209/44, C07D 265/30, C07D 207/46

(54) **Process for the preparation of nitroxyl radicals of sterically hindered amines**
Verfahren zur Herstellung von Nitroxylradikalen von gehinderten Aminen
Procédé pour la préparation de radicaux nitroxyl d'amines à empêchement stérique

(30) Priority: 30.11.1990 IT 2226490
(43) Date of publication of application: 03.06.1992
(73) Proprietor: GREAT LAKES CHEMICAL ITALIA S.r.l., I-20138 Milano (IT)
(72) Inventor: Neri, Carlo, Dr., I-20097 San Donato Milanese, Milan (IT); Costanzi, Sivestro, Dr., I-20090 Lodi Vecchio, Milan (IT); Riva, Rosa Maria, Dr., I-22055 Merate, Como (IT); Angaroni, Mariangela, Dr., I-21040 Geranzano, Varese (IT)
(74) Representative: Weinhold, Peter, Dr.

(56) References cited:
- DE-A- 1 695 738
- GB-A- 1 199 351

## Description

The present invention relates to a process for the preparation of stable nitroxyl radicals of sterically hindered amines.

It is known that these radicals are mainly used as stabilizers of organic polymers (P.P. Klemchuk, ACS Symposium, 1985); they are also used as spin labels which can be measured by means of radical detecting equipment.

There are basically two methods of sythesis known in the art, which can be used industrially. The first is based on the oxidation reaction of the above amines with hydrogen peroxide in the presence of tungsten containing catalysts. (see f.i. GB A 1199351)

The most suitable catalysts for this purpose are phosphotungstic acid and sodium tungstate.

The disadvantages of said method mainly reside in the impossibility of recycling the catalytic system and in the necessity of washing the reaction products at the end of the reaction to liberate them from the catalytic system which is liable to impair the activity thereof.

Another recent method for obtaining the nitroxyl derivatives of hindered amines (Tetrahedron Letters 29, (37), 4677 (1988)) comprises the reaction of said amines with dimethyldioxirane in the absence of catalysts. However, even if the yield of nitroxyl radicals is considerable, said process is quite costly due to the fact that a non-commercial product is used.

One object of the present invention, therefore, is to overcome the above-mentioned disadvantages of the known processes.

More specifically, the first aspect of the present invention is a new process for the preparation of nitroxyl radicals of sterically hindered amines, comprising the reaction of said amines with hydrogen peroxide in the presence of a titanium containing catalyst, e.g., titanium dioxide and titanium silicalites.

The present process is simple, economical and makes it possible to recover the catalyst at the end of the reaction and to possibly recycle it.

Originally titanium silicalites were considered excellent catalysts for the oxidation of olefinic and aromatic hydrocarbons of alkalis and phenols (Perego, Notari "Zeolites: Synthesis, Characterization and Application"; 1988; Catalytical Studies Division, 430 Ferguson Drive; Montain View, California).

Then there were also carried out oxidation reactions of aromatic amines in the presence of zeolites with the formation of diphenyl groups (J. Chem. Soc. Faraday Trans., 71, 1192-1202 (1975)) as well as oxidation reactions of aniline in the presence of titanium silicalites with the formation of azoxybenzene (A. Fujimura, M. Oguri, Y. Kano, M. Uemura, Cat. Symp. Acta, Hokkaido Univ. Japan, July 1989).

Also known is the oxidation reaction of secondary aliphatic amines with hydrogen peroxide in the presence of titanium silicalites with the formation of hydroxylamine derivatives (European Patent Appl. No. 88 117950.1).

In this case, the oxidation products of the secondary amines are hydroxylamines.

However, it has now surprisingly been found that by using sterically hindered amines as substrates, the reaction products are just those stable nitroxyl radicals which are used as stabilizers.

The oxidation reaction according to the present invention is effected, with high yields, in accordance with the following equation: 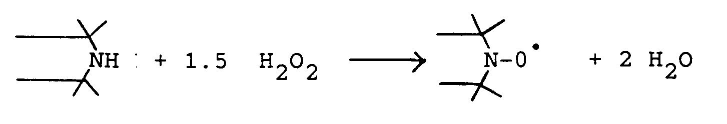
Preferred sterically hindered amines are those which comprise a (e.g. 5- or 6-membered) ring containing the structural unit -C(CH₃)₂-N-C(CH₃)₂-. Specific examples of such sterically hindered amines are: 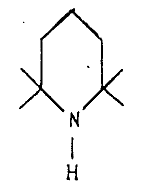
2,2,6,6-tetramethyl piperidine and its derivatives, e.g., 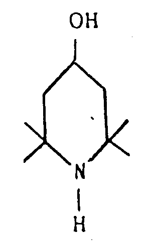
2,2,6,6-tetramethylpiperidine-4-ol 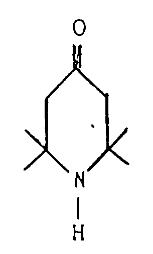
2,2,6,6-tetramethyl piperidine-4-one 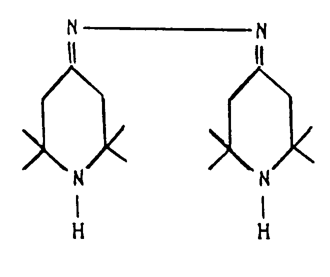
bis (2,2,6,6-tetramethyl-4-piperidinyl)-hydrazone 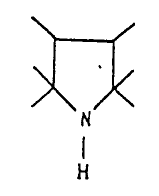
2,2,3,4,5,5-hexamethyl-pyrrolidine 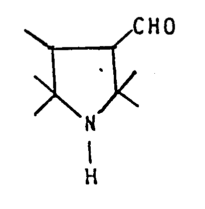
2,2,3,5,5-pentamethyl-4-formylpyrrolidine 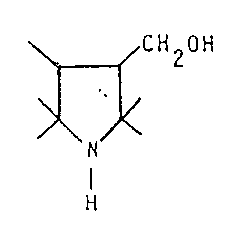
2,2,3,5,5-pentamethyl-4-hydroxymethylpyrrolidine 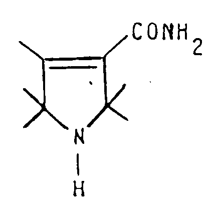
2,2,4,5,5-pentamethyl-3-pyrrolinyl carbamide 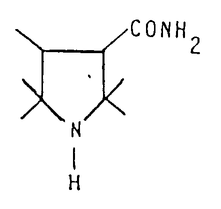
2,2,4,5,5-pentamethyl-3-pyrrolidinyl carbamide 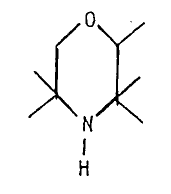
2,3,3,5,5-pentamethyl-morpholine 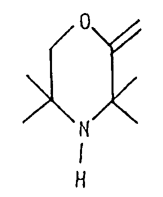
3,3,5,5-tetramethyl-2-methylenemorpholine 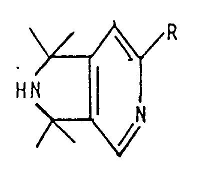
1,1,3,3-tetramethyl-pyrrolopyridine 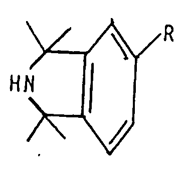
1,1,3,3-tetramethyl-2,3-dihydroisoindole where R is H or a linear or branched alkyl radical containing from 1 to 6 (e.g. 1 to 3) carbon atoms.

The synthesis of the nitroxyl radicals can be carried out by adding hydrogen peroxide to the mixture of catalyst and amine which is kept under stirring.

The reaction can be carried out with or without a solvent. If a solvent is used, both water and organic solvents can be employed.

Organic solvents suitable for this purpose are, e.g., ketones such as acetone and cyclohexanone, halogenated aliphatic hydrocarbons such as dichloroethane and methylene chloride and other similar products, and alcohols such as methanol, ethanol, propanol and (preferably tert.-)butanol.

It is also possible to use mixtures of the above alcohols and water.

The reaction is usually carried out at a temperature ranging from 20 to 100°C even if it is preferred to heat the reaction mixture to a temperature of from 40 to 80°C to accelerate the reaction and to improve the yield.

Preferably the molar quantity of hydrogen peroxide used in the oxidation reaction is from 2 to 4 times the molar quantity of the substrate.

The quantity of zeolitic catalyst normally used varies from 1 to 10% by weight of the weight of the amine even if, from the point of view of kinetics and cost of the catalyst, it is preferred to use quantities ranging from 4 to 7% of the weight of the amine.

As zeolitic synthetic catalyst containing titanium, it is convenient to use a titanium silicalite corresponding to the general formula

X TiO₂ (1-X) SiO₂

X ranging from 0.0001 to 0.04, preferably from 0.01 to 0.025.

This kind of catalyst and a method for its preparation are described in GB-B-2071071 (US-A-4410105).

Titanium silicalites with a defined structure are just as suitable as oxidation catalysts. Titanium silicalite with the ZSM-5 structure (Ti-ZSM-5) is particularly suitable.

The synthesis of titanium silicalite generally includes, however, the preparation of a reaction mixture containing sources of silicon oxide, titanium oxide and optionally sources of an alkali metal oxide, an organic nitrogen base and water, and having a composition, expressed in terms of the molar ratios of the reagents, within the following ranges:

| Reagents | Molar ratios | Preferred molar ratios |
|---|---|---|
| SiO₂/TiO₂ | 5 - 200 | 35 - 65 |
| OH/SiO₂ | 0.1 - 1.0 | 0.3 - 0.6 |
| H₂/SiO₂ | 20 - 200 | 60 - 100 |
| Me/SiO₂ | 0.0 - 0.5 | 0 |
| RN⁺/SiO₂ | 0.1 - 2.0 | 0.4 - 1.0 |

where Me represents an alkali metal ion, preferably Na or K, and RN⁺ represents the organic cation deriving from the organic nitrogen base.

The source of silicon oxide can be a tetralkyl-ortho-silicate, preferably tetraethyl-ortho-silicate, or quite simply colloidal silica, or the silicate of an alkali metal, preferably of Na or K.

The source of titanium oxide may be a hydrolyzable titanium compound, preferably chosen from TiCl₄, TiOCl₂ and Ti(alkoxy)₄, preferably Ti(OEt)₄.

A typical organic base is a tetra-alkylammonium hydroxide.

For the preparation of the titanium silicalite, the mixture of reagents is usually subjected to a hydrothermal treatment in an autoclave at temperatures ranging from 130 to 200°C, under autogenous pressure and over a period of 6 to 30 days, until the crystals of the precursor of the titanium-silicalite have formed; the latter are then separated from the mother liquor, thoroughly washed with water and dried; the precursor of the titanium-silicalite thus obtained, and having in its anhydrous state the following composition:

xTiO₂ (1-x)SiO₂ 0.04 (RN⁺)₂O

is heated in air, at approximately 550°C, for 1 to 72 hours, in order to completely eliminate the organic nitrogen base. The titanium-silicalite thus obtained has the above composition.

At the end of the reaction according to the present invention, the catalyst may easily be removed, e.g., by filtration, and, as stated previously, can be used again in other reactions with a slight loss of activity (causing a decrease in the rate of the reaction).

The yields obtained are generally high and depend on the kind of substrate and its sterical hindrance.

The reaction time usually varies from 3 to 24 hours.

At the end of the reaction, after the catalyst has been removed by filtration, the product may be recovered by extraction with an organic solvent and then usually purified by crystallization.

The following examples are intended to provide a clearer illustration of the present invention without limiting it in any way.

### EXAMPLE 1

Preparation of the 2,2,6,6-tetramethyl-piperidinyl-N-oxide radical of formula 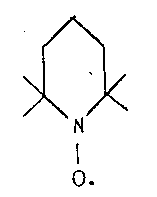
10 g (0.071 moles) of 2,2,6,6-tetramethyl piperidine, 0.7 g of titanium silicalite (TS-1) obtained as described in example 1 of GB-B-2071071 and 30 ml of methanol are placed in a flask equipped with thermometer, magnetic stirrer, reflux condenser and dropping funnel.

The mixture, under continuous stirring, is brought to the reflux temperature of the solvent (65°C) and a 30% w/w aqueous solution of H₂O₂ (32.2 g, 0.284 moles) is slowly added over a period of 1 hour.

The mixture is kept, under stirring, at a temperature of 65°C for a further 6 hours, the solution being analysed by gas-chromatography every hour.

When the reagent is no longer present, the solution is cooled and the catalyst removed by filtration.

NaCl is added to the filtered solution which is then extracted with two 20 ml portions of petroleum ether (boiling point 58-70°C).

The ether extracts are combined, dried over Na₂SO₄ and evaporated to remove the solvent.

A small quantity of hexane is then added to the residue which is left to crystallize at a temperature of 0°C.

The crystalline product in needle form, filtered at room temperature, washed with a small quantity (5 to 10 ml) of cold hexane and then dried under vacuum, has a bright red colour and a melting point of between 36 and 38°C.

Using the above procedure, 10.5 g (94.6% yield) of the product were recovered with the following elemental analysis:

| | C (%) | H (%) | N (%) |
|---|---|---|---|
| Theoretical | 69.2 | 11.6 | 8.96 |
| Experimental | 69.05 | 11.55 | 9.04 |

### EXAMPLES 2 TO 6

### Preparation of the 2,2,6,6-tetramethylpiperidinyl-N-oxide radical

The following examples demonstrate how the activity of the catalyst varies when it is reused in further reactions.

The same procedure as in example 1 is used and the catalyst is recycled after filtration.

Table 1 below shows the percentage of conversion of 2,2,6,6-tetramethyl-piperidine after 6 hours of reaction at the reflux temperature of methanol.

**TABLE 1**

| Example No. | Number of recycling operations | Conversion (%) of 2,2,6,6-tetramethylpiperidine after 6 h of reaction |
|---|---|---|
| 2 | 0 | 98 |
| 3 | 1 | 92 |
| 4 | 2 | 87 |
| 5 | 3 | 80 |
| 6 | 4 | 65 |

### EXAMPLES 7 TO 9

### Preparation of the 2,2,6,6-tetramethylpiperidinyl-N-oxide radical

The following examples show how the conversion varies in relation to the solvents used.

The same procedure as in example 1 is used, replacing the methanol by an equal amount (30 ml) of water (Example 7), water-acetone 1:1 (Example 8) and tert-butanol (Example 9).

Table 2 below shows the conversion after 6 hours of reaction determined by gas-chromatography.

**TABLE 2**

| Example No. | T (°C) | Conversion (%) after 6 h of reaction |
|---|---|---|
| 7 | 80 | 97 |
| 8 | 60 | 99 |
| 9 | 80 | 95 |

### EXAMPLE 10

### Preparation of the 2,2,6,6-tetramethyl-piperidine-4-ol-N-oxide radical of formula

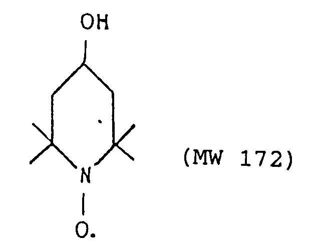

15.7 g (0.1 moles) of 2,2,6,6-tetramethylpiperidine-4-ol, 0.8 g of titanium silicalite (TS-1) and 40 ml of solvent consisting of a 1:1 by volume mixture of water-methanol, are placed in a flask equipped with thermometer, magnetic stirrer, reflux condenser and dropping funnel.

The mixture is heated to 70°C and 23 g (0.4 m) of 60% (w/w) aqueous H₂O₂ are then added over a period of 3 hours.

The reaction mixture is left for 24 hours at said temperature whereafter the solution is analysed by gas-chromatography.

Upon filtering off the catalyst, K₂CO₃ is added to the filtrate which is then extracted with diethyl ether.

The ether extract is dried over Na₂SO₄ and then evaporated.

Hot hexane is added to the residue which is then separated from the 2,2,6,6-tetramethyl-piperidine-4-ol, crystallized from the hexane, filtered and dried.

The product thus obtained in 95% yield, is in the form of red crystals having a melting point of 72 - 73°C.

### EXAMPLES 11 TO 16

Using the same procedure as in Example 10, nitroxyl radicals are obtained which are shown in Table 3 with the corresponding yields (in %) after reaction in a solvent composed of a 1:1 by weight mixture of water and methanol at a temperature of 70°C.

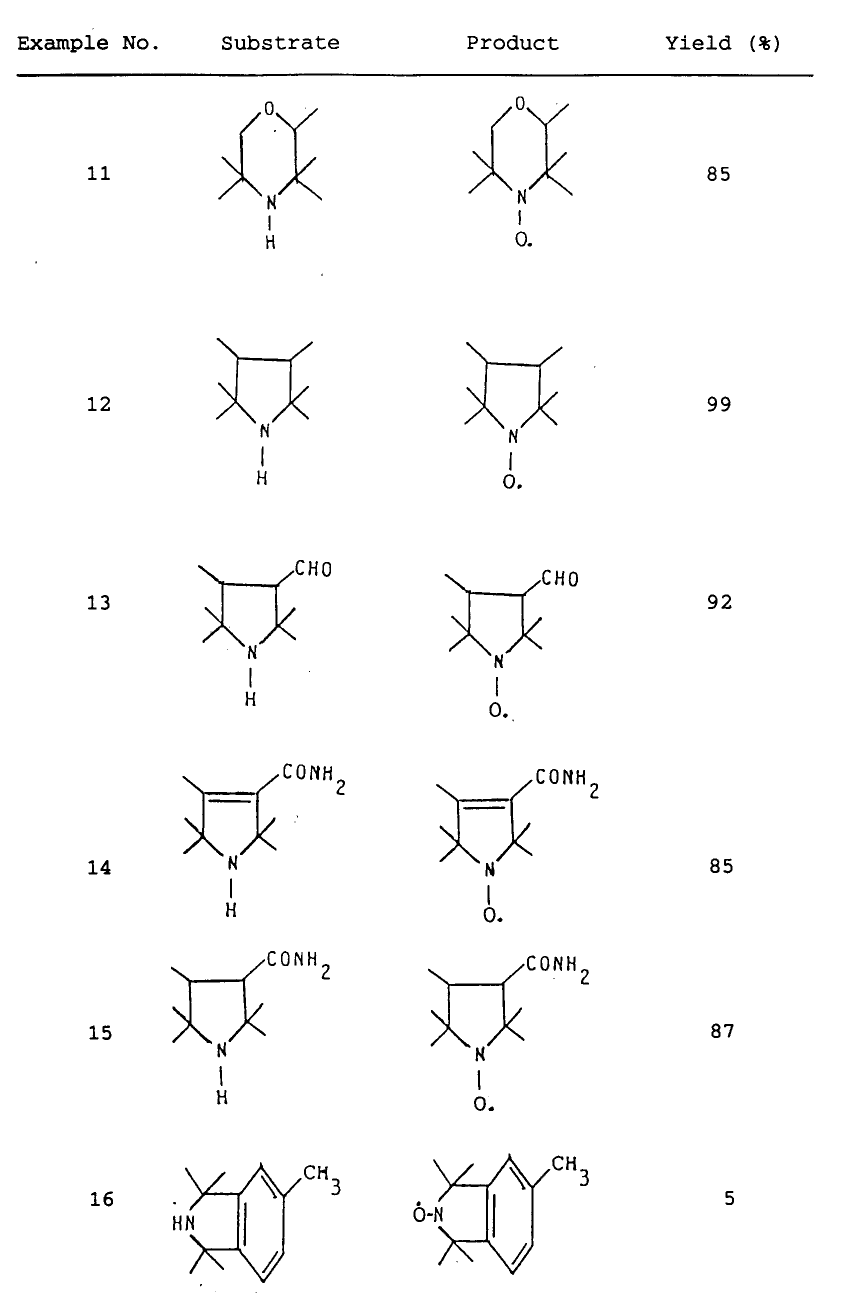

## Claims

1. Process for the preparation of nitroxyl radicals of sterically hindered amines, comprising the reaction of said amines with hydrogen peroxide in the presence of a titanium containing catalyst.

2. Process according to claim 1, wherein the titanium containing catalyst is a titanium silicalite.

3. Process according to claim 1, wherein the titanium containing catalyst is a synthetic zeolite corresponding to the general formula
X.TiO₂(1-X)SiO₂
X being a number of from 0.0001 to 0.04, particularly of from 0.01 to 0.025.

4. Process according to any one of the preceding claims, wherein the sterically hindered amine is selected from 2,2,6,6-tetramethyl-piperidine, 2,2,6,6-tetramethylpiperidine-4-ol, 2,2,6,6-tetramethylpiperidine-4-one, bis(2,2,6,6,-tetramethyl-4-piperidinyl)-hydrazone, 2,2,3,4,5,5-hexamethyl-pyrrolidine, 2,2,3,5,5-pentamethyl-4-formylpyrrolidine, 2,2,3,5,5-pentamethyl-4-hydroxymethylpyrrolidine, 2,2,3,5,5-pentamethyl-4-pyrrolidinyl carbamide, 2,2,3,5,5-pentamethyl-4-pyrrolinyl carbamide, 2,3,3,5,5-pentamethyl-morpholine, 3,3,5,5-tetramethyl-2-methylene-morpholine, 1,1,3,3-tetramethyl-pyrrolopyridine of formula 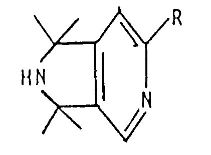 and 1,1,3,3,tetramethyl-2-dihydro-isoindole of formula 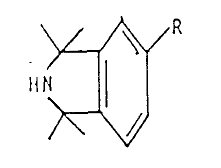 where R is H or a linear or branched alkyl radical containing from 1 to 6 carbon atoms.

5. Process according to any one of the preceding claims, wherein the reaction is carried out in the presence or absence of solvent.

6. Process according to any one of the preceding claims, wherein water and/or an organic solvent is used as solvent.

7. Process according to claim 6, wherein the solvent is selected from ketones such as acetone and cyclohexanone, halogenated aliphatic hydrocarbons such as dichloroethane and methylene chloride, alcohols such as methanol, ethanol, propanol and butanol, and alcohol-water mixtures.

8. Process according to any one of the preceding claims, comprising the addition, under continuous stirring, of hydrogen peroxide to a mixture of catalyst, amine, and solvent, if any, the stirring of the resulting mixture for about 3 to about 24 hours at a temperature of from 20 to 100°C, the removal of the catalyst and the recovery of the desired product by extraction with an organic solvent.

9. Process according to any one of the preceding claims, wherein the reaction is carried out at a temperature of from 40 to 80°C.

10. Process according to any one of the preceding claims, wherein the molar ratio of hydrogen peroxide to amine ranges from 2:1 to 4:1 and/or the weight ratio of catalyst to amine is from 0.01:1 to 0.1:1.

## Patentansprüche

1. Verfahren zur Herstellung von Nitroxylradikalen sterisch gehinderter Amine, welches die Umsetzung des Amins mit Wasserstoffperoxid in Gegenwart eines titanhaltigen Katalysators umfaßt.

2. Verfahren gemäß Anspruch 1, worin der titanhaltige Katalysator ein Titansilicalit ist.

3. Verfahren gemäß Anspruch 1, worin der titanhaltige Katalysator ein synthetischer Zeolith ist, der der allgemeinen Formel
X.TiO₂(1-X)SiO₂
entspricht, in der X eine Zahl von 0.0001 bis 0.04, insbesondere von 0.01 bis 0.025, ist.

4. Verfahren gemäß einem der vorhergehenden Ansprüche, worin das sterisch gehinderte Amin ausgewählt ist aus 2,2,6,6-Tetramethylpiperidin, 2,2,6,6-Tetramethylpiperidin-4-ol, 2,2,6,6-Tetramethylpiperidin-4-on, Bis(2,2,6,6-tetramethyl-4-piperidinyl)-hydrazon, 2,2,3,4,5,5-Hexamethylpyrrolidin, 2,2,3,5,5-Pentamethyl-4-formylpyrrolidin, 2,2,3,5,5-Pentamethyl-4-hydroxymethypyrrolidin, 2,2,3,5,5-Pentamethyl-4-pyrrolidinyl-carbamid, 2,2,3,5,5-Pentamethyl-4-pyrrolinyl-carbamid, 2,2,3,5,5-Pentamethylmorpholin, 3,3,5,5-Tetramethyl-2-methylenmorpholin, 1,1,3,3-Tetramethylpyrrolopyridin der Formel 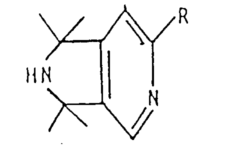 und 1,1,3,3-Tetramethyl-2-dihydroisoindol der Formel 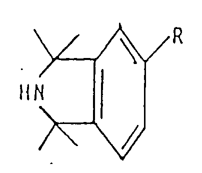 in denen R gleich H oder ein linearer oder verzweigter Alkylrest mit 1 bis 6 Kohlenstoffatomen ist.

5. Verfahren gemäß irgendeinem der vorhergehenden Ansprüche, worin die Reaktion in Anwesenheit oder Abwesenheit von Lösemittel durchgeführt wird.

6. Verfahren gemäß irgendeinem der vorhergehenden Ansprüche, worin Wasser und/oder ein organisches Lösemittel als Lösemittel verwendet werden.

7. Verfahren gemäß Anspruch 6, worin das Lösemittel ausgewählt ist aus Ketonen wie Aceton und Cyclohexanon, halogenierten aliphatischen Kohlenwasserstoffen wie Dichlorethan und Methylenchlorid, Alkoholen wie Methanol, Ethanol, Propanol und Butanol und Alkohol-Wasser-Mischungen.

8. Verfahren gemäß irgendeinem der vorhergehenden Ansprüche, umfassend die Zugabe von Wasserstoffperoxid unter ständigem Rühren zu einer Mischung von Katalysator, Amin und Lösemittel, falls vorhanden, Rühren der resultierenden Mischung für etwa 3 bis etwa 24 Stunden bei einer Temperatur von 20 bis 100 °C, Entfernen des Katalysators und Gewinnen des gewünschten Produkts durch Extraktion mit einem organischen Lösemittel.

9. Verfahren gemäß irgendeinem der vorhergehenden Ansprüche, worin die Umsetzung bei einer Temperatur von 40 bis 80 °C durchgeführt wird.

10. Verfahren gemäß irgendeinem der vorhergehenden Ansprüche, worin das Molverhältnis von Wasserstoffperoxid zu Amin im Bereich von 2 : 1 bis 4 : 1 und/oder das Gewichtsverhältnis von Katalysator zu Amin zwischen 0.01 : 1 und 0.1 : 1 liegt.

## Revendications

1. Procédé de préparation de radicaux N-oxyde d'amines à empêchement stérique, comprenant la réaction desdites amines avec du peroxyde d'hydrogène en présence d'un catalyseur renfermant du titane.

2. Procédé selon la revendication 1, dans lequel le catalyseur renfermant du titane est une silicalite au titane.

3. Procédé selon la revendication 1, dans lequel le catalyseur renfermant du titane est une zéolite synthétique répondant à la formule générale :
X.TiO₂(1-X)SiO₂
dans laquelle X représente un nombre compris dans l'intervalle allant de 0,0001 à 0,04, en particulier un nombre compris dans l'intervalle allant de 0,01 à 0,025.

4. Procédé selon l'une quelconque des revendications précédentes, dans lequel l'amine à empêchement stérique est choisie parmi la 2,2,6,6-tétraméthylpipéridine, le 2,2,6,6-tétraméthylpipéridine-4-ol, la 2,2,6,6-tétraméthylpipéridine-4-one, la bis(2,2,6,6-tétraméthyl-4-pipéridinyl)hydrazone, la 2,2,3,4,5,5-hexaméthylpyrrolidine, la 2,2,3,5,5-pentaméthyl-4-formylpyrrolidine, la 2,2,3,5,5-pentaméthyl-4-hydroxyméthylpyrrolidine, le 2,2,3,5,5-pentaméthyl-4-pyrrolidinylcarboxamide, le 2,2,3,5,5-pentaméthyl-4-pyrrolinylcarboxamide, la 2,3,3,5,5-pentaméthylmorpholine, la 3,3,5,5-tétraméthyl-2-méthylènemorpholine, les 1,1,3,3-tétraméthyl-pyrrolopyridines de formule : 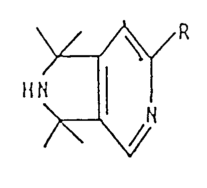 et les 1,1,3,3-tétraméthyl-2,3-dihydroisoindoles de formule : 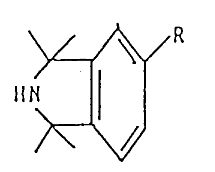 R représentant un atome d'hydrogène ou un groupe alkyle, linéaire ou ramifié, contenant 1 à 6 atomes de carbone.

5. Procédé selon l'une quelconque des revendications précédentes, dans lequel on réalise la réaction en présence ou en l'absence d'un solvant.

6. Procédé selon l'une quelconque des revendications précédentes, dans lequel on utilise comme solvant, de l'eau et/ou un solvant organique.

7. Procédé selon la revendication 6, dans lequel on choisit le solvant parmi des cétones telles que l'acétone et la cyclohexanone, des hydrocarbures aliphatiques halogénés tels que le dichloroéthane et le chlorure de méthylène, des alcools tels que le méthanol, l'éthanol, le propanol et le butanol, et les mélanges alcool-eau.

8. Procédé selon l'une quelconque des revendications précédentes, comprenant l'addition, sous agitation continue, de peroxyde d'hydrogène à un mélange de catalyseur, d'amine et éventuellement de solvant, l'agitation du mélange résultant pendant environ 3 heures à environ 24 heures, à une température de 20°C à 100°C, l'élimination du catalyseur et la récupération du produit désiré par extraction à l'aide d'un solvant organique.

9. Procédé selon l'une quelconque des revendications précédentes, dans lequel on réalise la réaction à une température comprise dans l'intervalle allant de 40 à 80°C.

10. Procédé selon l'une quelconque des revendications précédentes, dans lequel le rapport molaire du peroxyde d'hydrogène à l'amine est compris dans l'intervalle allant de 2:1 à 4:1, et/ou le rapport en poids du catalyseur à l'amine est compris dans l'intervalle allant de 0,01:1 à 0,1:1.
